(19) Europäisches Patentamt
European Patent Office
Office européen des brevets

(11) Veröffentlichungsnummer: 0 298 440
A1

(12) # EUROPÄISCHE PATENTANMELDUNG

(21) Anmeldenummer: 88110774.2

(51) Int. Cl.4: C07D 401/12 , A61K 31/415

(22) Anmeldetag: 06.07.88

Patentansprüche für folgende Vertragsstaaten: ES + GR.

(30) Priorität: 10.07.87 DE 3722810

(43) Veröffentlichungstag der Anmeldung: 11.01.89 Patentblatt 89/02

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Lang, Hans-Jochen, Dr.
Rüdesheimer Strasse 7
D-6238 Hofheim am Taunus(DE)
Erfinder: Weidmann, Klaus, Dr.
Talweg 11
D-6242 Kronberg/Taunus(DE)
Erfinder: Herling, Andreas W., Dr.
Am Walberstück 5
D-6277 Bad Camberg(DE)

(54) **Substituierte Benzimidazole, Verfahren zu deren Herstellung, diese enthaltende pharmazeutische Zubereitungen und deren Verwendung.**

(57) Die Erfindung betrifft Verbindungen der Formel

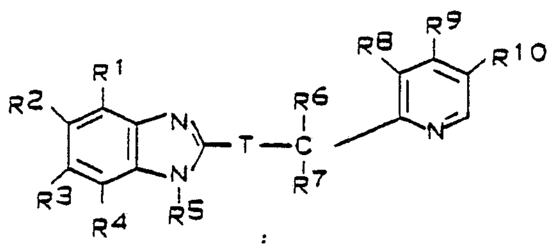

(I)

in welcher T -S-, -SO- oder -$SO_2$-, bedeutet, und $R^1$, $R^2$, $R^3$, $R^4$, $R^5$, $R^6$, $R^7$, $R^8$, $R^9$ und $R^{10}$ die in der Beschreibung definierten Bedeutungen haben, Verfahren zu deren Herstellung, dieser enthaltende pharmazeutische Zubereitungen und deren Verwendung.

EP 0 298 440 A1

# Substituierte Benzimidazole, Verfahren zu deren Herstellung, diese enthaltende pharmazeutische Zubereitungen und deren Verwendung

Benzimidazol-Derivate mit magensäuresekretionshemmender Wirkung sind beispielsweise aus der EP-A-184 322 und der EP-A-197 013 bekannt.

Es wurden neue, hochwirksame Magensäuresekretionshemmer der Formel I gefunden,

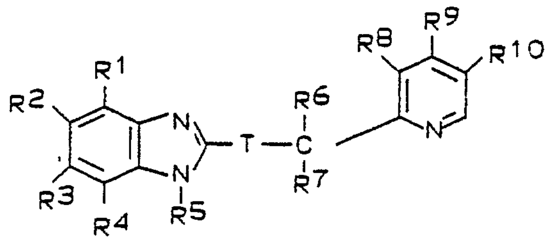

(I)

in welcher
T -S-, -SO- oder $-SO_2-$, vorzugsweise -SO- bedeutet,
$R^1$, $R^2$, $R^3$ und $R^4$ gleich oder verschieden sind und Wasserstoff, Halogen, Cyano, Nitro, $(C_1-C_6)$-Alkyl, einen halogenierten Alkylrest $(C_fH_{2f+1-g})Hal_g$, $(C_1-C_6)$-Hydroxyalkyl, $(C_1-C_6)$-Alkoxy, einen halogenierten Alkoxyrest $OC_fH_{(2f+1-g)}Hal_g$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder zwei der Reste $R^1$, $R^2$, $R^3$ und $R^4$ die benachbart sind, gemeinsam für $-[CQ_2]_n-$ oder -CH=CH-CH=CH- stehen, worin eine oder zwei $CQ_2$-Gruppen durch gleiche oder verschiedene Reste aus der Reihe O, S, SO und $SO_2$ ersetzt sein können, und die übrigen der Reste $R^1$, $R^2$, $R^3$ und $R^4$ vorstehende Bedeutung haben;
$R^5$ Wasserstoff, $(C_1-C_{11})$-Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet;
$R^6$ und $R^7$ gleich oder verschieden sind und Wasserstoff oder $(C_1-C_3)$-Alkyl bedeuten;
$R^8$ und $R^{10}$ gleich oder verschieden sind und Wasserstoff, Halogen, $(C_1-C_{12})$-Alkyl, Trifluormethyl, $(C_3-C_6)$-Cycloalkyl, $(C_1-C_{12})$-Alkoxy, $-O-CH_2-C_fH_{(2f+1-g)}F_g$, $-NR'R''$, $(C_1-C_{12})$-Alkoxy-$(C_1-C_{12})$-alkyl, $(C_1-C_{12})$ Alkoxy-$(C_1-C_{12})$-alkoxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeuten, mit der Maßgabe, daß mindestens einer der Reste $R^8$ und $R^{10}$ Halogen bedeuten muß;
$R^9$ $(C_6-C_{12})$-Alkoxy, $(C_3-C_8)$-cycloalkyloxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeutet;
Hal Halogen bedeutet;
Q Wasserstoff oder Halogen bedeutet;
$R'$ und $R''$ gleich oder verschieden sind und Wasserstoff oder $(C_1-C_4)$-Alkyl bedeuten oder
$R'$ und $R''$ gemeinsam für $-[CH_2]_h-$ stehen, worin eine $CH_2$-Gruppe durch O, S, N-$(C_1-C_4)$-Alkanoylimino oder N-$(C_1-C_4)$-Alkoxycarbonylimino ersetzt sein kann;
f = 1, 2, 3, 4, 5 oder 6, vorzugsweise 1-4 ist;
g = 1 bis (2f+1) ist;
h = 4, 5 oder 6 ist;
und
n = 3 oder 4 ist,
sowie deren physiologisch verträglichen Salze.

Alkyl und davon abgeleitete Reste wie beispielsweise Alkoxy, Alkylmercapto, Alkylsulfinyl, Alkylsulfonyl, Aralkyl oder Alkanoyl können geradkettig oder verzweigt sein.

$(C_6-C_{12})$-Aryl ist beispielsweise Phenyl, Naphthyl oder Biphenylyl, bevorzugt ist Phenyl.

$(C_7-C_{11})$-Aralkyl ist beispielsweise Benzyl oder Phenethyl, vorzugsweise Benzyl. Entsprechendes gilt für davon abgeleitete Reste wie Aralkyloxy.

Halogen steht für Fluor, Chlor, Brom oder Jod.

$R^5$ steht vorzugsweise für Wasserstoff, $(C_1-C_6)$-Alkylcarbamoyl oder einen Rest der Formel VI,

$-(CO-O-)_p\ (CR^{11}R^{12}-O-)_q\ W-B$ (VI)

worin p = 0 oder 1, q = 0 oder 1 und B Wasserstoff, einen Acylrest oder einen gegebenenfalls substituierten Alkylrest bedeuten. Besonders bevorzugt ist $R^5$ = Wasserstoff.

$R^{11}$ und $R^{12}$ sind gleich oder verschieden und bedeuten Wasserstoff, $(C_1-C_6)$-Alkyl, $(C_3-C_8)$-Cycloalkyl, $(C_7-C_{11})$-Aralkyl oder $(C_6-C_{12})$-Aryl.

B und $R^{11}$ können auch gemeinsam für eine $-[CH_2]_r$-Kette mit r = 3, 4 oder 5 - vorzugsweise 4 - stehen, wobei in einer oder mehreren der $CH_2$-Gruppen jeweils ein Wasserstoffatom durch OH, geschütztes OH, Amino, Acylamino und/oder Halogen ersetzt sein kann. Bei einem Rest mit substituierter $-[CH_2]_r$-Kette handelt es sich vorzugsweise um einen gegebenenfalls mit in der Kohlenhydratchemie üblichen Schutzgruppen teilweise oder vollständig geschützten Glycosylrest, der sich von einer Glycopyranose, Glycofuranose oder einem Oligosaccharid ableitet.

Der Glycosylrest kann sowohl α- als auch β-glycosidisch verknüpft sein.

Er kann beispielsweise einen Glucofuranosyl- oder Glucopyranosyl-Rest sein, der sich von natürlich vorkommenden Aldotetrosen, Aldopentosen, Aldohexosen, Ketopentosen, Desoxyaldosen, Aminoaldosen und Oligosacchariden, wie Di- und Trisacchariden, sowie deren Stereoisomeren ableiten.

Diese Glycosylreste leiten sich insbesondere von natürlichen, in Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetylgalactosamin (GalNAc), N-Acetyl-mannosamin (ManNAc) oder Disacchariden, wie Maltose (Mal), Lactose (Lac), Cellobiose (Cel), Gentibiose (Gen), N-Acetyl-lactosamin (LacNAc), Chitobiose (Chit), β-Galactopyranosyl-(1→ 3)-N-acetylgalactosamin und β-Galactopyranosyl-(1→ 3)- oder -(1→ 4)-N-acetyl-glucosamin, sowie deren synthetischen Derivate, wie 2-Desoxy-, 2-Amino-, 2-Acetamido- oder 2-Halogeno-, bevorzugt Bromo- oder Iodo-Zucker ab.

Unter den in der Kohlenhydratchemie üblichen Schutzgruppen versteht man z.B. die $(C_1-C_{10})$ Acylschutzgruppen wie $(C_1-C_6)$-Alkanoyl (z.B. Acetyl-, Trichloracetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-, Benzyliden-, Isopropyliden- oder Trityl-Gruppe, wobei hier die Acylschutzgruppen, insbesondere die Acetyl-(Ac)-Gruppe, bevorzugt sind.

a) Falls p = q = 0 ist, haben die Reste vorzugsweise folgende Bedeutungen:

W ist eine Bindung oder bedeutet -CO-, $-CR^{13}R^{14}-$ oder $-CO-CR^{13}R^{14}-$.

B bedeutet Wasserstoff (nur, falls W keine Bindung ist), $(C_1-C_{10})$-Alkyl; $(C_2-C_{12})$-Alkenyl; $(C_3-C_{12})$-Cycloalkyl; $(C_6-C_{12})$-Aryl, das gegebenenfalls durch 1, 2 oder 3 gleiche oder verschiedene Reste aus der Reihe $(C_1-C_4)$-Alkyl, Chlor, Brom, Fluor, Nitro, Trifluormethyl, $(C_1-C_4)$-Alkoxy und Hydroxy substituiert ist; $-(CH_2)_s-CH(NH_2)-R^{15}$ mit s = 1 - 9; den Acylrest einer Aminosäure oder $(C_1-C_6)$-Alkyl, das durch bis zu 4 gleiche oder verschiedene Reste aus der Reihe F, Cl oder Br substituiert ist.

$R^{13}$ und $R^{14}$ sind gleich oder verschieden und bedeuten Wasserstoff, $(C_1-C_6)$-Alkyl, $(C_1-C_6)$-Alkoxy, $(C_3-C_8)$-Cycloalkyl, $(C_7-C_{11})$Aralkyl, $(C_6-C_{12})$-Aryl oder Pyridyl oder $R^{13}$ und $R^{14}$ stehen gemeinsam für $-[CH_2]_4-$, $-[CH_2]_5-$ oder $[CH_2]_6-$, worin 1 oder 2 $CH_2$-Gruppen durch 0 ersetzt sein können.

$R^{15}$ bedeutet Wasserstoff oder $(C_1-C_{10})$-Alkyl.

b) Falls q = 1 ist, sind W und B wie oben unter (a) definiert. Darüber hinaus kann W -CO-O- und $-CO-O-CR^{13}-R^{14}-$ bedeuten, wobei $R^{13}$ und $R^{14}$ die obengenannten Bedeuturgen haben. B kann auch im Falle von W = Bindung für Wasserstoff stehen.

c) Falls p = 1 und q = 0 ist, steht W für eine Bindung oder bedeutet $-CR^{13}R^{14}-$ , wobei $R^{13}$ und $R^{14}$ die Bedeutungen wie unter (a) haben. B ist definiert wie unter (a), kann aber nicht für den Acylrest einer Aminosäure stehen. -CO-O-W-B kann darüber hinaus für weitere $N^{im}$-Schutzgruppen vom Urethantyp stehen, die von der vorstehenden Definition nicht umfaßt werden (vgl. z.B. Hubbuch, Kontakte Merck 3/79 14-23; Büllesbach, Kontakte Merck 1/80 23-35).

Unter einem gegebenenfalls substituierten $(C_6-C_{12})$-Arylrest (siehe oben unter (a)) wird beispielsweise Phenyl, (o-, m-, p-)Tolyl, (o-, m-, p-)Ethylphenyl, 2-Ethyl-tolyl, 4-Ethyl-o-tolyl, 5-Ethyl-m-tolyl, (o-, m-oder p-)Propylphenyl, 2-Propyl-(o-, m- oder p-)Tolyl, 4-Isopropyl-2,6-xylyl, 3-Propyl-4-ethylphenyl, (2,3,4-, 2,3,6-, oder 2,4,5-)Trimethylphenyl, (o-, m- oder p-)-Fluorphenyl, (o-, m- oder p-Trifluormethyl)phenyl, 4-Fluor-2,5-xylyl, (2,4-, 2,5-, 2,6-, 3,4- oder 3,5-)Difluorphenyl, (o-, m- oder p-)Chlorphenyl, 2-Chlor-p-tolyl, (3-, 4-, 5-oder 6-)Chlorotolyl, 4-Chlor-2-propylphenyl, 2-Isopropyl-4-chlorphenyl, 4-Chlor-3,5-xylyl, (2,3-, 2,4-, 2,5-, 2,6-oder 3,5-)Dichlorphenyl, 4-Chlor-3-fluorphenyl, (3- oder 4-)-Chlor-2-fluorphenyl, (o-, m- oder p-)Trifluormethylphenyl, (o-, m- oder p-)Ethoxyphenyl, (4- oder 5-)Chlor-2-methoxyphenyl, 2,4-Dichlor-(5- oder 6-)methylphenyl oder (o-, m- oder p )Methoxyphenyl verstanden.

$(C_1\text{-}C_{10})$-Alkyl ist beispielsweise Methyl, Ethyl, Propyl, Butyl, Pentyl, Hexyl, Heptyl, Octyl, Nonyl, Decyl oder deren isomere Formen.

$(C_3\text{-}C_{12})$-cycloalkyl schließt alkylsubstituiertes Cycloalkyl und bi- und mehrcyclische Systeme mit ein. Es wird darunter beispielsweise verstanden: Cyclopropyl, 2-methylcyclopropyl, 2,2-Dimethylcyclopropyl, 2,3-Diethylcyclopropyl, 2-Butylcyclopropyl, Cyclobutyl, 2-Methylcyclobutyl, 3-Propylcyclobutyl, 2,3,4-Triethylcyclobutyl, Cyclopentyl, 2,2-Dimethylcyclophenyl, 2-Pentylcyclopentyl, 3-tert-Butylcyclopentyl, 2,2-Dimethylcyclohexyl, Cycloheptyl, Cyclononyl, Cyclodecyl, Norbornyl oder Adamantyl.

Unter einem Acylrest einer Aminosäure wird vorzugsweise der Rest einer α-Aminosäure, insbesondere aus der Reihe der natürlich vorkommenden α-Aminosäuren oder deren Antipoden verstanden, wie z.B. H-Gly-, H-Ala-, H-Val-, H-Leu-, H-Ile, H-Phe-, H-Lys-, H-Pro-, H-Trp-, H-Met-, H-Ser-, H-Thr-, H-Cys-, H-Tyr-, H-Asn-, H-Gln-, H-Asp-, H-Glu-, H-Arg-, H-Orn- oder die entsprechenden Reste in der D-Konfiguration.

Ohne daß der Erfindungsgegenstand darauf beschränkt wäre, seien im folgenden einige erfindungsgemäße Urethanschutzgruppen $R^5$ = -CO-O-WB genannte: $(C_1\text{-}C_6)$-Alkoxycarbonyl wie Boc; $(C_3\text{-}C_{12})$-Cycloalkyloxycarbonyl wie Mboc, Iboc oder Adoc;

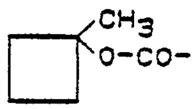

Mboc

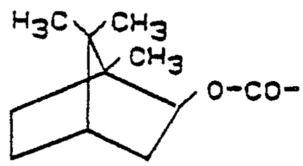

Iboc

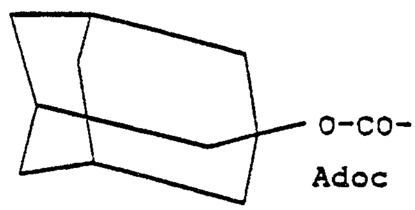

Adoc

$(C_3\text{-}C_{12})$-Cycloalkyl-$(C_1\text{-}C_6)$-alkoxycarbonyl wie Adpoc;

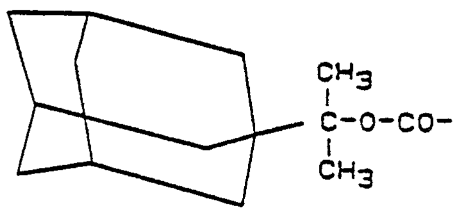

Adpoc

$(C_6\text{-}C_{12})$-Aryl-$(C_1\text{-}C_6)$-alkoxycarbonyl wie Z, Fmoc oder Bpoc,

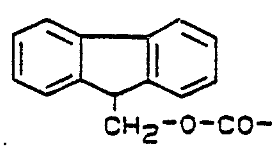

Fmoc

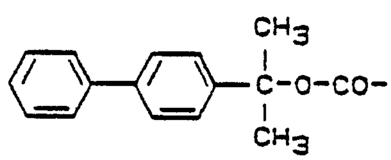

Bpoc

substituierte Z-Reste wie Moc, Ddz und Z (p-$NO_2$)

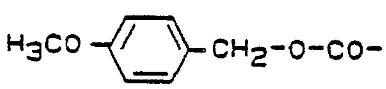

Moc

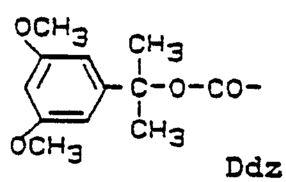

Ddz

und modifizierte Z-Reste wie Pyoc und deren von 2- bzw. 3-Picolin abgeleitete Reste, die oben bei $(C_6\text{-}C_{12})$-Aryl angegeben substituiert sein können.

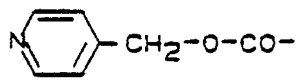

Pyoc

Bevorzugte $N^{im}$-Schutzgruppen sind solche, die in Gegenwart von Säuren, vorzugsweise in einem pH-Bereich von etwa 1 - 6 und/oder unter physiologischen Bedingungen abgespalten werden können.

Durch gezielte Auswahl einer $N^{im}$-Schutzgruppe ist es somit für den Fachmann möglich, die Freisetzung der aktiven Verbindungen so zu steuern, daß diese selektiv am Wirkort erfolgt.

Halogenierte Alkylreste $C_fH_{(2f+1-g)}Hal_g$ sind beispielsweise durch 1 bis 8 Fluoratome substituierte ($C_1$-$C_6$)-Alkylreste wie Trifluormethy, 2,2,2-Trifluorethyl, 2,2,3,3,3-Pentafluorpropyl, 1-(Trifluormethyl 2,2,2-trifluorethyl, 2,2,3,3-Tetrafluorpropyl, 2,2,3,3,4,4,4-Heptafluorbutyl und 2,2,3,3,4,4,5,5-Octafluorpentyl. Entsprechendes gilt für die davon abgeleiteten Alkoxyreste, wobei insbesondere durch 3 bis 5 Fluoratome substituierte Ethoxyreste geeignet sind, wie 2,2,2-Trifluorethoxy, 1,1,2-Trifluorethoxy, 1,2,2-Trifluorethoxy, 1,2,2,2-Tetrafluorethoxy, Perfluorethoxy vorzugsweise 1,1,2,2-Tetrafluorethoxy.

Stehen zwei der Reste $R^1$, $R^2$, $R^3$ und $R^4$ für $-[CQ_2]_n-$, worin $CQ_2$-Gruppen durch andere Gruppen ersetzt sind, so sind beispielsweise $-O-[CQ_2]_{n-2}-O-$ Reste geeignet, wie Methylendioxy, Difluormethylendioxy, Ethylendioxy oder Trifluorethylendioxy.

Gegebenenfalls in den erfindungsgemäßen Verbindungen der Formel I vorhandene chirale C- und S-Atome können sowohl in der R- als auch in der S-Konfiguration vorkommen. In solchen Fällen liegen Verbindungen der Formel I in Form der reinen Enantiomeren oder als Stereoisomerengemisch (wie Enantiomerengemisch und Diasteromerengemisch) vor.

Als Salze kommen insbesondere Alkali- und Erdalkalisalze und Salze mit physiologisch verträglichen Aminen in Frage.

Verbindungen der Formel I sind bevorzugt, worin $R^8$ und $R^{10}$ gleich oder verschieden sind und Wasserstoff, Halogen oder ($C_1$-$C_6$)-Alkyl bedeuten, wobei mindestens einer dieser Reste Halogen bedeuten muß.

Insbesondere sind solche Verbindungen der Formel I bevorzugt, worin T eine -SO-Gruppe bedeutet,
$R^2$, $R^3$, $R^6$ und $R^7$ jeweils Wasserstoff bedeuten;
$R^9$ ($C_1$-$C_{12}$)-Alkoxy, $O-CH_2-C_fH_{(2f+1-g)}F_g$, ($C_1$-$C_{12}$)-Alkoxy-($C_1$-$C_{12}$)-alkyl, ($C_1$-$C_{12}$)-Alkoxy-($C_1$-$C_{12}$)-alkoxy, ($C_7$-$C_{11}$)-Aralkyloxy, ($C_1$-$C_{12}$)-Alkylmercapto, ($C_1$-$C_{12}$)-Alkylsulfinyl oder ($C_1$-$C_{12}$) Alkylsulfonyl bedeuten;
$R^8$ und $R^{10}$ gleich oder verschieden sind und Wasserstoff, Halogen oder ($C_1$-$C_6$)-Alkyl bedeuten, wobei mindestens einer der Reste $R^8$ und $R^{10}$ Halogen bedeuten muß.

Die Erfindung betrifft auch ein Verfahren zur Herstellung von Verbindungen der Formel I, das dadurch gekennzeichnet ist, daß man

a) eine Verbindung der Formel II

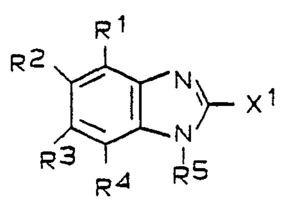

(II)

in welcher $R^1$, $R^2$, $R^3$, $R^4$ und $R^5$ wie im Anspruch 1 definiert sind und
$X^1$ i. eine Abgangsgruppe oder
ii. -SH, $-S^-$ oder $-SO_2^-$ bedeutet,
umsetzt mit einer Verbindung der Formel III

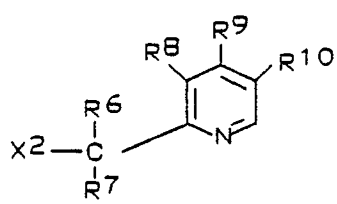

(III)

in welcher $R^6$, $R^7$, $R^8$, $R^9$ und $R^{10}$ wie im Anspruch 1 definiert sind und
$X^2$ im oben genannten Fall i. -SH, $-S^-$ oder $-SO_2^-$ und
im oben genannten Fall ii. eine Abgangsgruppe bedeutet, oder
b) eine Verbindung der Formel IV,

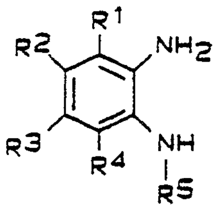
(IV)

in welcher $R^1$, $R^2$, $R^3$, $R^4$ und $R^5$ wie oben definiert sind, umsetzt mit einer Verbindung der Formel V

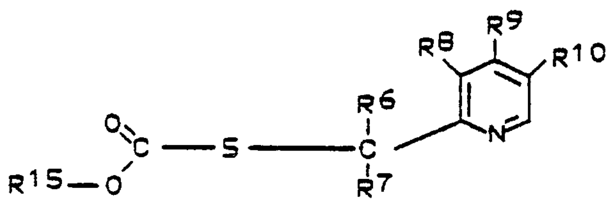
(V)

in welcher $R^6$, $R^7$, $R^8$, $R^9$ und $R^{10}$ wie oben definiert sind und $R^{15}$ für eine veresternde Gruppe steht, oder
c) eine Verbindung der Formel VI

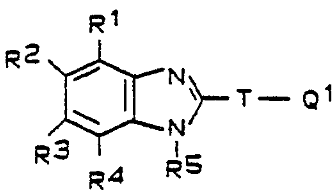
(VI)

in welcher $R^1$, $R^2$, $R^3$, $R^4$ und T wie oben definiert sind, $R^5$ Wasserstoff oder eine Schutzgruppe bedeutet und
$Q^1$ a) für $CR^6R^7$-M steht oder
b) eine Abgangsgruppe bedeutet,
umsetzt mit einer Verbindung der Formel VII

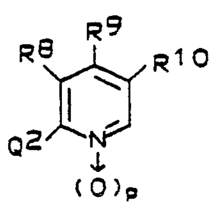
(VII)

in welcher $R^8$, $R^9$ und $R^{10}$ wie oben definiert sind und
$Q^2$ a) falls $Q^1$ wie oben unter a) definiert ist, eine Abgangsgruppe bedeutet, oder
b) falls $Q^1$ wie oben unter b) definiert ist, für $CR^6R^7$-M steht, und
p = 0 oder 1 ist,
wobei in den Formeln VI und VII $R^6$ und $R^7$ wie oben definiert ist und
M für ein Alkalimetallatom oder dessen Äquivalent steht
i. in einer Verbindung der Formel I (eine) gegebenenfalls vorhandene -S-Gruppe(n) gewünschtenfalls zu(r) -SO-oder $-SO_2$-Gruppe(n) oxidiert,

ii. in einer Verbindung der Formel I (eine) gegebenenfalls vorhandene -SO-Gruppe(n) gewünschtenfalls zu(r) $-SO_2$-Gruppe(n) oxidiert,

iii. eine Verbindung der Formel I, worin $R^5$ für Wasserstoff steht, gewünschtenfalls acyliert, alkyliert oder aralkyliert,

iv. eine Verbindung der Formel I, worin $R^5$ nicht Wasserstoff bedeutet, gewünschtenfalls verseift und

v. eine Verbindung der Formel I gewünschtenfalls in ihre physiologisch verträglichen Salze überführt,

wobei zwei oder mehr der Maßnahmen i.-iv. auch in einer anderen als der angegebenen Reihenfolge ausgeführt werden können.

Setzt man gemäß der hier bevorzugten Verfahrensvariante (a) Verbindungen der Formel II mit Verbindungen der Formel III um, so steht $X^1$ oder $X^2$ für eine Abgangsgruppe, die nucleophil ablösbar ist, wie Cl, Br, I, $-O-SO_2-CH_3$, $-O-SO_2-CF_3$ oder $-O-SO_2-(C_6H_4-pCH_3)$. Eine Abgangsgruppe $X^2$ kann darüberhinaus beispielsweise $(C_1-C_4)$-Acyloxy, vorzugsweise $(C_1-C_4)$-Alkanoyloxy wie Acetoxy, oder Hydroxy bedeuten.

Die Umsetzung einer Verbindung der Formel II mit einer Verbindung der Formel III oder deren Salzen erfolgt in einem inerten Lösungsmittel wie z.B. Wasser, Methylenchlorid, Methanol, Ethanol, Aceton, Essigsäureethylester, Toluol, Tetrahydrofuran, Acetonitril, Dimethylformamid, Dimethylsulfoxid oder Gemischen dieser Lösungsmittel zweckmäßigerweise in Gegenwart einer anorganischen oder organischen Base, wie z.B. Natrium- oder Kaliumhydroxid, -carbonat, -alkoxid, -hydrid, -amid, Ammoniak, Triethylamin, Tributylamin, Pyridin bei -20 bis +150 °C, vorzugsweise bei 0 bis 80 °C.

In den bei Verfahrensvariante (b) eingesetzten Estern der Formel V steht $R^{15}$ für eine veresternde Gruppe, vorzugsweise $(C_1-C_6)$-Alkyl oder Benzyl.

Die Umsetzung einer Verbindung der Formel IV mit einer Verbindung der Formel V gemäß Verfahrensvariante (b) erfolgt analog der in Preston et al., Benzimidazoles and Congeneric Tricyclic Compounds, Part 1, New York, Seiten 10-13 beschriebenen Verfahrensweisen.

Bei Verfahrensvariante (c) sind als Alkalimetallatome M beispielsweise Lithium, Natrium oder Kalium geeignet. Unter ihrem Äquivalent versteht man beispielsweise Erdalkaliatome wie beispielsweise Magnesium, die durch ein Halogenatom wie Chlor, Brom oder Iod substituiert sind. T ist vorzugsweise -SO-. $R^6$ und $R^7$ sind vorzugsweise jeweils Wasserstoff.

Eine geeignete Abgangsgruppe $Q^2$ ist beispielsweise Halogen, vorzugsweise Chlor, oder Hydroxy, das durch Veresterung mit einer Aryl- oder Alkylsulfonsäure aktiviert ist. Als Sulfonsäure kommen beispielsweise p-Toluolsulfonsäure oder Methansulfonsäure in Frage.

Als Abgangsgruppe $Q^1$ eignen sich vorzugsweise jene, die mit der hier bevorzugten -SO-Gruppe ein reaktives Sulfinsäurederivat bilden, wie $(C_1-C_4)$-Alkoxy, Di-$(C_1-C_4)$-alkylamino und $(C_1-C_4)$-Alkylmercapto.

Verfahrensvariante (c) kann in einem inerten organischen Lösungsmittel zwischen -20 °C und dem Siedepunkt des Reaktionsgemisches, vorzugsweise zwischen Raumtemperatur und dem Siedepunkt des Reaktionsgemisches durchgeführt werden. Die Wahl der geeigneten Reaktionstemperatur hängt ab von der Natur der Gruppen $Q^1$ und $Q^2$.

Falls $Q^1$ für $CR^6R^7M$ steht, sind beispielsweise jene Lösungsmittel geeignet, in denen üblicherweise Enolate mit Alkylierungsmitteln umgesetzt werden, wie z.B. Benzol oder Toluol. Falls $Q^2$ für $CR^6R^7M$ steht, eignen sich die Lösungsmittel, in denen gewöhnlich organometallische Reaktionen durchgeführt werden, wie niedrige aliphatische Ether (z.B. Diethylether) oder THF.

Die so erhaltenen Verbindungen der Formel I können, falls $R^5$ Wasserstoff bedeutet, in physiologisch verträgliche Salze umgewandelt werden.

Verbindungen der Formel I mit T = -S- können ferner mit geeigneten Oxidationsmitteln in solcher mit T = -SO- oder $-SO_2$- umgewandelt werden. In gleicher Weise lassen sich auch -S-Gruppen in den Substituenten $R^1$ bis $R^4$ und $R^8$ bis $R^{10}$ oxidieren.

Diese Reaktion erfolgt in einem geeigneten, inerten Lösungsmittel wie z.B. Methylenchlorid, Chloroform, Tetrachlorkohlenstoff, 1,2-Dichlorethan, Toluol, Essigsäureethylester, Essigsäure, Trifluoressigsäure, Wasser, Methanol, Ethanol oder Gemischen derselben bei -20 °C bis +150 °C, vorzugsweise bei -10 °C bis +40 °C.

Als Oxidationsmittel kommen z.B. in Betracht: Wasserstoffperoxid, Persäuren und Perester wie Peressigsäure, Trifluorperessigsäure, Monoperphthalsäure, m-Chlorperbenzoesäure und deren Ester, Ozon, Distickstofftetroxid, Iodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, Natrium-meta-perjodat, Selen- oder Mangandioxid, Cerammonnitrat, Chromsäure, Chlor, Brom, Diazabicyclo-[2.2.2]octan-Bromkomplex, Dioxandibromid, Pyridiniumbromidperbromid, Sulfurylchlorid, 2-Arylsulfonyl-3-aryloxaziridine, Titantetraisopropylat/tert. Butylhydroperoxid (gegebenenfalls unter Zusatz von Dialkylestern der (D)- bzw. (L)-Weinsäure und einer definierten Menge Wasser.

Ebenso können isolierte, ggf. immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5 - 10 Mol-% bei der Oxidation zu T = -SO- oder auch in größerem Überschuß und/oder bei höherer Reaktionstemperatur eingesetzt wenn eine Oxidation zu T = $-SO_2-$ gewünscht wird.

Verbindungen der Formel I mit $R^5 \neq H$ können hergestellt werden ausgehend von Verbindungen der Formel IV mit $R^5 \neq H$ und Verbindungen der Formel V oder durch Acylierung, Alkylierung oder Aralkylierung von Verbindungen der Formel I mit $R^5$ = H. Im folgenden soll auf den zweiten Weg etwas näher eingegangen werden.

Die Acylierung, Alkylierung oder Aralkylierung von Verbindungen der Formel I wird in an sich bekannter Weise mit den entsprechenden Acylierungsmitteln, Alkylierungsmitteln bzw. Aralkylierungsmitteln in einem geeigneten organischen Lösungsmittel in der Regel bei einer Temperatur zwischen -78 °C und dem Siedepunkt des Reaktionsgemisches gegebenenfalls in Gegenwart einer Base durchgeführt.

$N^{im}$-Schutzgruppen der Formel VI mit p = 0, q = 1, W = Bindung und B = Wasserstoff lassen sich in Verbindungen der Formel I ($R^5$ = H, T = S) beispielsweise durch Hydroxyalkylierung einführen, wobei man $N^{im}$-Schutzgruppen mit $R^{11}$ = $R^{12}$ = Wasserstoff in an sich bekannter Weise (vgl. z.B. Eur. J. Med. Chem. 15 [1980] 586; J. Med. Chem. 22 [1979] 1113) durch Hydroxymethylierung mit Formaldehyd in einem organischen Lösungsmittel wie z.B. Acetonitril einführen kann. Die Hydroxyalkylierung wird bei einer Temperatur zwischen 0 °C und dem Siedepunkt des Reaktionsgemisches gegebenenfalls in Gegenwart einer Base wie Triethylamin durchgeführt.

Die Zwischenprodukte der Formel IV und die intermediären Benzimidazol-Derivate der Formeln II und VI sind bekannt oder können in Analogie zu bekannten Verfahren hergestellt werden. Z.B. können Verbindungen der Formel II, worin $X^1$ SH bedeutet, durch Umsetzung der entsprechenden Verbindungen der Formel IV mit Schwefelkohlenstoff in Gegenwart von Alkalimetallhydroxiden, mit Kaliumethylxanthogenat (Org. Synth. 30 56) oder Thiophosgen hergestellt werden. Verbindungen der Formel II, worin $X^1$ eine Abgangsgruppe wie Halogen oder Tosylat bedeutet, können aus den entsprechenden Verbindungen der Formel II, worin $X^1$ Hydroxy bedeutet, durch Behandeln mit geeigneten Reagenzien wie z.B. $POCl_3$ hergestellt werden. Die Verbindungen der Formel II, worin $X^1$ Hydroxy bedeutet, werden erhalten durch Behandeln von Verbindungen der Formel IV mit Phosgen. Verbindungen der Formel IV können in Analogie zu den in EP-A-127 763, DE-A-28 48 53, Chem. Abstr. 60 [1964] 13352h und Liebigs Ann. Chem. 730 - [1969] 16-30 beschriebenen Methoden hergestellt werden.

Verbindungen der Formel III, worin $X^2$ Hydroxy und $R^6$ und $R^7$ jeweils Wasserstoff bedeuten, können aus Verbindungen der Formel VIII

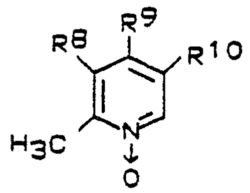

VIII

worin $R^8$, $R^9$ und $R^{10}$ wie oben definiert sind, durch Umlagerung beispielsweise mit Essigsäureanhydrid oder Trifluoressigsäureanhydrid hergestellt werden.

Wenn man Verbindungen der Formel III, worin $X^2$ Hydroxy bedeutet, mit einem Halogenierungsmittel, wie Thionylchlorid oder einem eine andere nucleophil ablösbare Abgangsgruppe übertragenden Reagenz wie z.B. p-Toluolsulfonylchlorid behandelt, erhält man Verbindungen der Formel III, in welcher $X^2$ eine nucleophil ablösbare Abgangsgruppe bedeutet.

Verbindungen der Formel III, worin $X^2$ für SH steht, lassen sich beispielsweise aus Verbindungen der Formel III, worin $X^2$ eine Abgangsgruppe wie Halogen bedeutet, durch Behandeln mit einem Hydrogensulfid wie NaSH herstellen.

Verbindungen der Formel V können beispielsweise aus dem Mercaptiden der Verbindungen der Formel III, in welcher $X^2$ SH bedeutet, durch Umsetzung mit den entsprechenden Chlorameisensäureestern hergestellt werden.

Verbindungen der Formel VII können im Analogie zu den in Roszniki Chem. 35 [1961] 475 beschriebenen Verfahren hergestellt werden.

Die Zwischenprodukte der Formel III und V können in Analogie zu bekannten Verfahren hergestellt werden, wie sie beispielsweise in "The Chemistry of Heterocyclic Compounds-Pyridine and its Derivatives", Teilbände 2 und 3, E. Klingsberg Ed., Interscience Publishers, 1962 beschrieben werden.

So lassen sich Verbindungen der Formel III, in welcher $X^2$ Hydroxy bedeutet, beispielsweise durch folgende Reaktionssequenz herstellen:

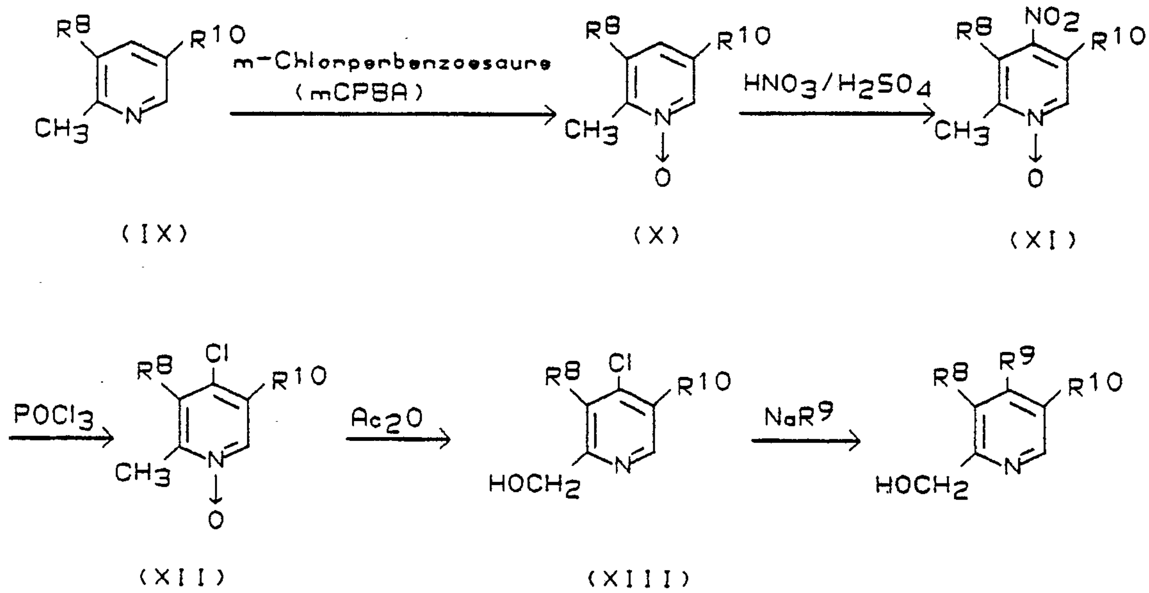


(IX) (X) (XI)

(XII) (XIII)

Verbindungen der Formel IX, worin $R^8$ oder $R^{10}$ Halogen bedeutet, sind literaturbekannt oder können nach literaturbekannten verfahren hergestellt werden.

Mittels folgender Reaktionssequenz lassen sich beispielsweise Verbindungen der Formel III herstellen, worin $X^2$ hydroxy, $R^8$ Chlor und $R^{10}$ Wasserstoff oder ($C_1$-$C_{12}$)-Alkyl bedeuten.

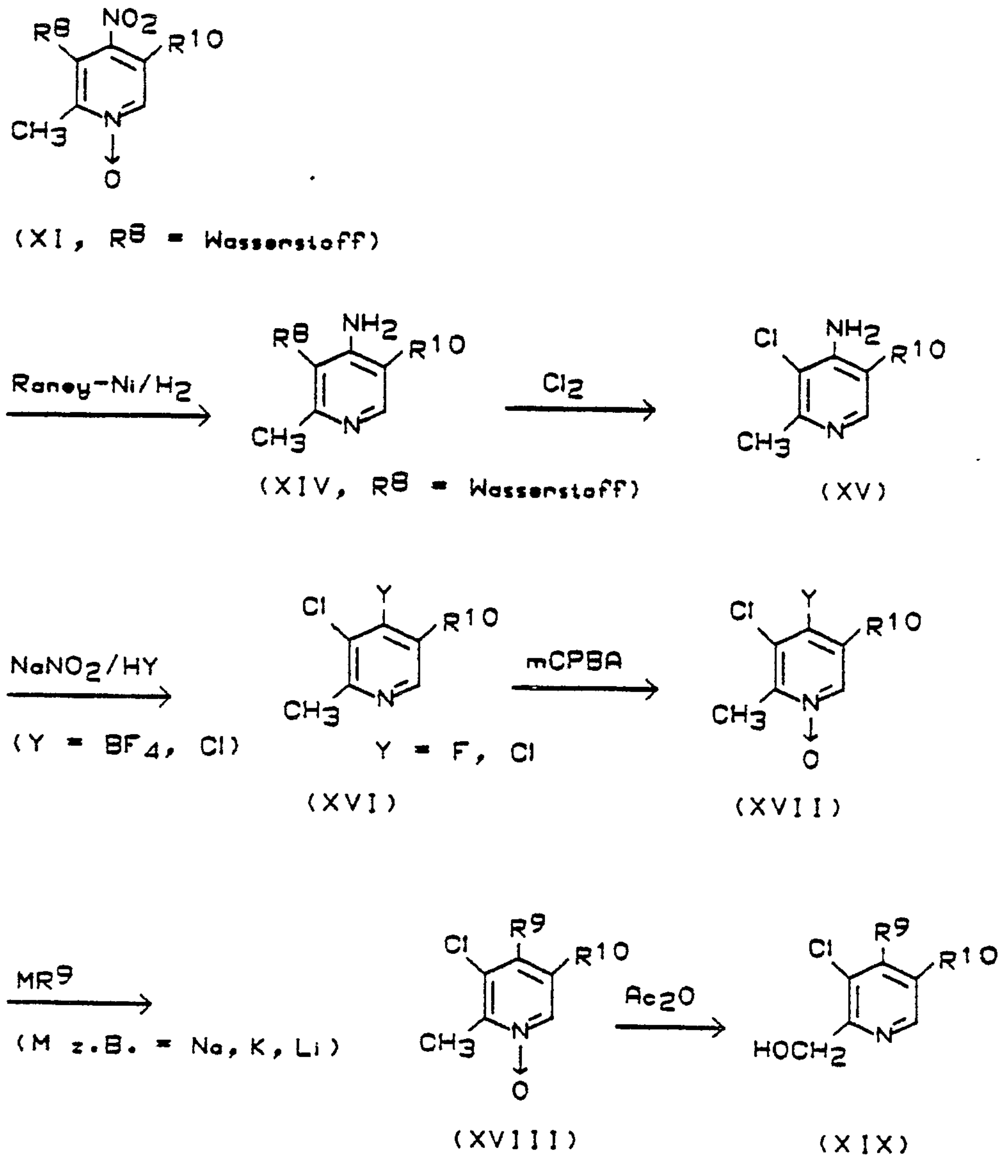


(XI, $R^8$ = Wasserstoff)

(XIV, $R^8$ = Wasserstoff)

(XV)

(XVI)

(XVII)

(XVIII)

(XIX)

Verbindungen der formel III mit $X^2$ = Cl, in denen einer der Substituenten $R^8$ und $R^{10}$ Chlor oder Brom und der andere Alkyl bedeutet, können auch über folgende Synthesewege hergestellt werden:

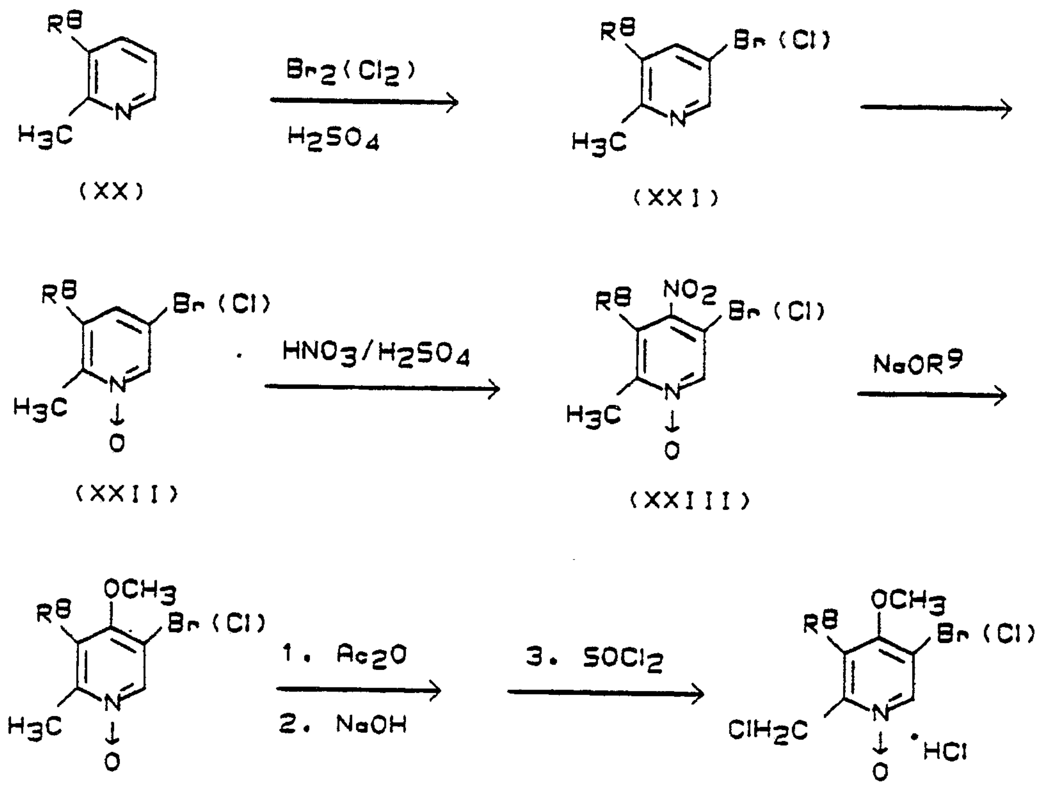


(XX) (XXI) (XXII) (XXIII) (XXIV) (XXV)

oder

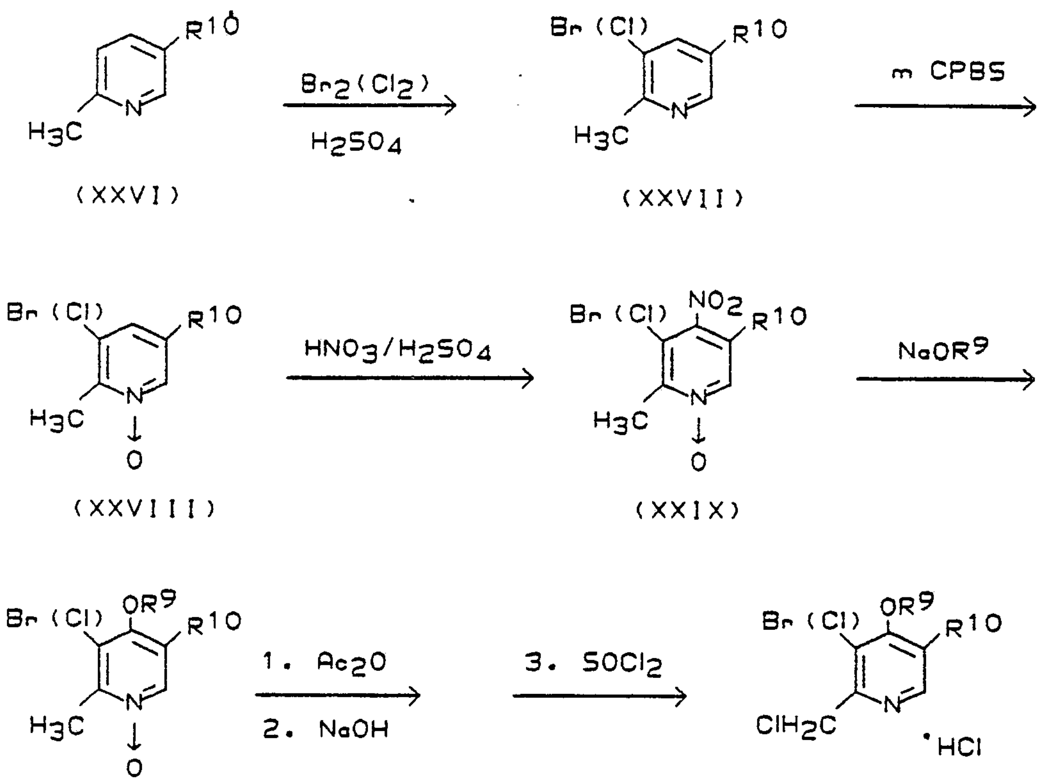


(XXVI) (XXVII) (XXVIII) (XXIX) (XXX) (XXXI)

Die Herstellung der Verbindung XXVII ($R^{10}$ = $CH_3$) durch Bromierung von 2,5-Dimethyl-pyridin ist literaturbekannt (siehe Abblard et al. Bull. Soc. Chim. France 1972, Nr. 6, 2430).

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Benzimidazole der Formel I und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Es wurde gefunden, daß auch die Colon-$K^+$-ATPase (vgl. Gustin, Goodman, J. Biol. Chem. 256 [1981] 10651-10656) in vitro stark durch Verbindungen gehemmt wird, die beim Behandeln der erfindungsgemäßen Verbindungen der Formel I mit Säure (z.B. mit Natriumacetat/Hcl-Puffer mit einem pH-wert von etwa 4-5,5) entstehen. Solche Umwandlungsprodukte können sich auch in vivo bei der Passage der Verbindungen der Formel I durch den Magen-Darm-Trakt bilden. In welchem Umfang sie gebildet werden, hängt vom Substitutionsmuster und vom pH ab.

Der Colon-$K^+$-ATPase wird ein entscheidender Einfluß auf das Elektrolytgleichgewicht an der Darmschleimhaut zugeschrieben. Colon-$K^+$-ATpase-Hemmer, wie die oben genannten, können daher in dieses Gleichgewicht eingreifen und zur Behandlung von Krankheiten mit gestörtem Elektrolytgleichgewicht dienen.

Die Erfindung betrifft daher auch die Verwendung von Verbindungen der Formel I bzw. deren Säure-Umwandlungsprodukte bei der Behandlung von Durchfallerkrankungen. Beispiele solcher Krankheiten sind entzündliche Darmerkrankungen, wie Cholera, Paratyphus, Reisediarrhoe oder andere Formen der sekretorischen Diarrhoe aber auch Colitis ulcerosa und regionale Enteritis.

Die Erfindung betrifft weiterhin Umwandlungsprodukte, die beim Behandeln von Verbindungen der Formel I mit Säure gebildet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 95 % beträgt.

Welche Hilfsstoffe für die gewünschte Arzneimittelformulierungen geeignet sind, ist dem Fachmann aufgrund seines Wissens geläufig. Neben Lösemitteln, Gelbildern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Applikation können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten

Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaestetika, wie z.B. Tetracain, Procain; gegebenenfalls auch Gastrinantaionisten, Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung als Trocken- oder als Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern, ohne die Erfindung auf die hier stellvertretend genannten Substanzen zu beschränken.

Die angegebenen Schmelz- und Zersetzungspunkte sind nicht korrigiert oder standardisiert.

**Beispiel 1a:**

3-Chlor-4-methoxy-2-picolin-N-oxid:

Zu einer Natriummethylatlösung, dargestellt aus 0,51 g Natrium und 20 ml Methanol, gibt man bei -10 °C 3,5 g 3,4-Dichlor-2-picolin-N-oxid in 20 ml wasserfreiem Methanol. Man läßt langsam auf Raumtemperatur erwärmen und erhitzt sodann 1 Stunde am Rückfluß. Nun destilliert man das Lösungsmittel unter vermindertem Druck ab, versetzt den Rückstand mit Wasser, extrahiert mit Dichlormethan und vertreibt das Lösungsmittel.
Farblose Kristalle aus Diisopropylether, Fp. 94 - 97 °C.

**Beispiel 1b:**

3-Chlor-2-hydroxymethyl-4-methoxypyridin:

5,8 g 3-Chlor-4-methoxy-2-picolin-N-oxid werden in 8 ml Eisessig gelöst und unter Rühren bei 90 °C mit 14 ml Acetanhydrid versetzt. Man erhitzt 2 Stunden auf 110-115 °C läßt sodann auf 80 °C abkühlen und tropft 25 ml Methanol zu. Anschließend wird unter vermindertem Druck das Lösungsmittel abdestilliert, der Rückstand sodann mit 20 ml Wasser und 8 g Ätznatron in kleinen Portionen versetzt und diese Mischung 2 Stunden zum Rückfluß erhitzt. Nach dem Abkühlen extrahiert man mit Dichlormethan, vertreibt das Lösungsmittel und bringt den Rückstand mit Diethylether zur Kristallisation.
Feststoff, Fp. 103-105 °C.

**Beispiel 1c:**

3-Chlor-2-chlormethyl-4-methoxypyridin-Hydrochlorid:

Zu einer Mischung aus 2,6 g 3-Chlor-2-hydroxymethyl-4-methoxypyridin und 30 ml Dichlormethan tropft man bei -10 bis -15 °C eine Lösung aus 3,5 ml Thionylchlorid in 25 ml Dichlormethan und rührt sodann 2 Stunden bei Raumtemperatur Man vertreibt das Lösungsmittel und bringt den Rückstand mit Diethylether zur Kristallisation.
Farblose Kristalle, Fp. 145-146 °C.

**Beispiel 1d:**

2-(3-Chlor-4-methoxy-2-picolylmercapto)-5-methoxy-1H-benzimidazol

Zu 0,45 g 5-Methoxy-2-mercapto-benzimidazol in 12 ml Ethanol werden 6,2 ml 2N wäßrige NaOH gegeben und bei -10 °C unter Rühren portionsweise 0,55 g 3-Chlor-2-chlormethyl-4-methoxypyridin-Hydrochlorid hinzugefügt. Man läßt auf Raumtemperatur erwärmen, rührt 1 Stunde, engt im Vakuum ein, nimmt den Rückstand in $CH_2Cl_2$ auf und wäscht mit Wasser. Nach Trocknen der organischen Phase über $MgSO_4$ wird vom Lösungsmittel befreit und der Rückstand mit Essigester an Kieselgel chromatographiert. Das aus entsprechenden Fraktionen erhaltene Rohprodukt wird aus Diethylether umkristallisiert.
Farblose Kristalle, Fp. 161-163 °C.

**Beispiel 2:**

2-(3-Chlor-4-methoxy-2-picolylsulfinyl)-5-methoxy-1H-benzimidazol

0,42 g der Titelverbindung aus Bsp. 1d werden in 15 ml Dichlormethan gelöst und mit 20 ml einer wäßrigen Pufferlösung aus $KH_2PO_4/Na_2HPO_4$; pH = 7,5 versetzt. Dann tropft man bei Raumtemperatur 0,25 g m-Dichlorperbenzoesäure, gelöst in Dichlormethan, zu.
Nach Abtrennen der organischen Phase, Trocknen über $MgSO_4$ und Abdestillieren des Lösungsmittels wird der Rückstand mit Diethylether zur Kristallisation gebracht.
Fp. 162-164 °C.

**Beispiel 3:**

2-(3-Chlor-4-methoxy-2-picolylmercapto)-5-trifluormethyl-1H-benzimidazol

Die Titelverbindung wird analog Beispiel 1d ausgehend von 5-Trifluormethyl-2-mercapto-benzimidazol und 3-Chlor-4-methoxy-2-chlormethylpyridin-Hydrochlorid hergestellt. Der ölige Rückstand kristallisiert beim Versetzen mit Wasser.
Fp. 108 °C (Zers.).

**Beispiel 4:**

2-(3-Chlor-4-methoxy-2-picolylsulfinyl-5-trifluormethyl-1H-benzimidazol

Die Titelverbindung erhält man analog Beispiel 2 aus der Titelverbindung von Beispiel 3.
Fp. 162 °C, (Zers.).

**Beispiel 5:**

3-Chlor-4-methoxy-5-methyl-2-picolin-N-oxid

10,6 g 3,4-Dichlor-2,5-dimethylpyridin-N-oxid werden unter Erwärmen in 250 ml wasserfreiem Methanol gelöst und bei 10 °C zu einer Natriummethylatlösung, dargestellt aus 1,44 g Natrium und 80 ml Methanol hinzugetropft. Anschließend erhitzt man 3 Stunden zum Rückfluß, gibt weitere Natriummethylatlösung (1,0 g Natrium in 40 ml Methanol) hinzu, erhitzt weitere 3 Stunden am Rückfluß. Nun destilliert man das Lösungsmittel unter vermindertem Druck ab, versetzt den Rückstand mit Wasser, extrahiert mit Dichlormethan und vertreibt das Lösungsmittel.
Farblose Kristalle aus Diisopropylether, Fp. 112-114 °C. In analoger Weise wurden hergestellt:

**Beispiel 6:**

2-(3-Chlor-4-methoxy-2-picolylmercapto)-5-methoxy-1H-benzimidazol

Fp. 113 °C (Zers.).

**Beispiel 7:**

2-(5-Brom-4-methoxy-3-methyl-2-picolylmercapto)-5-methoxy-1H-benzimidazol-dihydrochlorid

Fp. 197 °C (Zers.).

**Beispiel 8:**

2-(5Brom-4-methoxy-3-methyl-2-picolylsulfinyl)-5-methoxy-1H-benzimidazol

Fp. 175-176 °C.

**Ansprüche**

1. Verbindung der Formel I

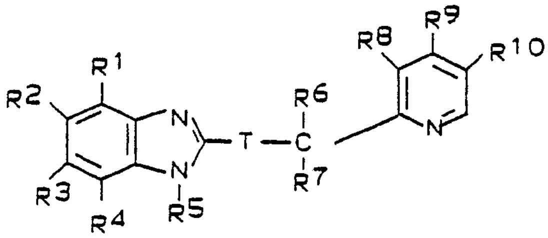

(I)

in welcher
T -S-, -SO- oder $-SO_2-$ bedeutet,
$R^1$, $R^2$, $R^3$ und $R^4$ gleich oder verschieden sind und Wasserstoff, Halogen, Cyano, Nitro, $(C_1-C_6)$-Alkyl, einen halogenierten Alkylrest $(C_fH_{2f+1-g})Hal_g$, $(C_1-C_6)$-Hydroxyalkyl, $(C_1-C_6)$-Alkoxy, einen halogenierten Alkoxyrest $OC_fH(2f+1-g)Hal_g$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder zwei der Reste $R^1$, $R^2$, $R^3$ und $R^4$ die benachbart sind, gemeinsam für $-[CQ_2]_n-$ oder -CH=CH-CH=CH- stehen, worin eine oder zwei $CQ_2$-Gruppen durch gleiche oder verschiedene Reste aus der Reihe O, S, SO und $SO_2$ ersetzt sein können, und die übrigen der Reste $R^1$, $R^2$, $R^3$ und $R^4$ vorstehende Bedeutung haben;
$R^5$ Wasserstoff $(C_1-C_{11})$-Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet;
$R^6$ und $R^7$ gleich oder verschieden sind und Wasserstoff oder $(C_1-C_3)$-Alkyl bedeuten;
$R^8$ und $R^{10}$ gleich oder verschieden sind und Wasserstoff, Halogen, $(C_1-C_{12})$-Alkyl, Trifluormethyl, $(C_3-C_6)$-Cycloalkyl, $(C_1-C_{12})$-Alkoxy, $-O-CH_2-C_fH_{(2f+1-g)}F_g$, $-NR'R''$, $(C_1-C_{12})$-Alkoxy-$(C_1-C_{12})$-alkyl, $(C_1-C_{12})$-Alkoxy-$(C_1-C_{12})$-Alkoxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeuten, mit der Maßgabe, daß mindestens einer der Reste $R^8$ und $R^{10}$ Halogen bedeuten muß;
$R^9$ $(C_5-C_{12})$-Alkoxy, $(C_3-C_8)$-Cycloalkyloxy, $(C_7-C_{11})$-Aralkyloxy, $(C_1-C_{12})$-Alkylmercapto, $(C_1-C_{12})$-Alkylsulfinyl oder $(C_1-C_{12})$-Alkylsulfonyl bedeutet;
Hal Halogen bedeutet;
Q Wasserstoff oder Halogen bedeutet;
$R'$ und $R''$ gleich oder verschieden sind und Wasserstoff oder $(C_1-C_4)$-Alkyl bedeuten oder
$R'$ und $R''$ gemeinsam für $-[CH_2]_h-$ stehen, worin eine $CH_2$-Gruppe durch O, S, N-$(C_1-C_4)$-Alkanoylimino oder N-$(C_1-C_4)$-Alkoxycarbonylimino ersetzt sein kann;
f = 1, 2, 3, 4, 5 oder 6, vorzugsweise 1-4 ist;
g = 1 bis (2f+1) ist;
h = 4, 5 oder 6 ist
und
n = 3 oder 4 ist,
sowie deren physiologisch verträglichen Salze.

2. Verbindungen der Formel I gemäß Anspruch 1, in welcher T für -SO- steht und deren physiologisch verträglichen Salze.

3. Verbindung der Formel I gemäß einem oder mehrerer der Ansprüche 1 bis 2, in welcher $R^8$ und $R^{10}$ gleich oder verschieden sind und Wasserstoff, Halogen oder $(C_1-C_5)$-Alkyl bedeuten, wobei mindestens einer dieser Reste Halogen bedeuten muß, und deren physiologisch verträglichen Salze.

4. Verbindung der Formel I gemäß einem oder mehrerer der Ansprüche 1 bis 3, in welcher
T eine -SO-Gruppe bedeutet
$R^2$, $R^3$, $R^6$ und $R^7$ jeweils Wasserstoff bedeuten;
$R^9$ $(C_1-C_{12})$-Alkoxy, $-O-CH_2-C_fH_{(2f+1-g)}F_g$, $(C_1-C_{12})$-Alkoxy-$(C_1-C_{12})$-alkyl, $(C_1-C_{12})$-Alkoxy-$(C_1-C_{12})$-alkoxy, $(C_7-C_{11})$-Aralkyloxy,$(C_1-C_{12})$-Alkylmercapto,$(C_1-C_{12})$-Alkylsulfinyl oder $(C_1-C_{12})$-Alkylsulfonyl bedeuten;
$R^8$ und $R^{10}$ gleich oder verschieden sind und Wasserstoff, Halogen oder $(C_1-C_6)$-Alkyl bedeuten, wobei mindestens einer der Reste $R^8$ und $R^{10}$ Halogen bedeuten muß, und deren physiologisch verträglichen Salze.

5. Verbindung der Formel I gemäß einem oder mehrerer der Ansprüche 1 bis 5, in welcher $R^5$ für Wasserstoff steht, und deren physiologisch verträglichen Salze.

6. Verfahren zur Herstellung einer Verbindung der Formel I gemäß einem der Ansprüche 1 bis 5, dadurch gekennzeichnet, daß man

a) eine Verbindung der Formel II

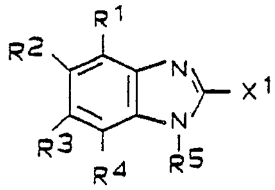

(II)

in welcher $R^1$, $R^2$, $R^3$, $R^4$ und $R^5$ wie im Anspruch 1 definiert sind und $X^1$ i. eine Abgangsgruppe oder

ii. -SH, $-S^-$ oder $-SO_2^-$ bedeutet,

umsetzt mit einer Verbindung der Formel III

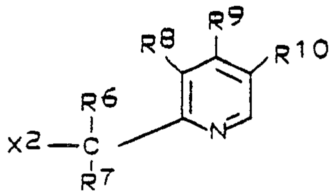

(III)

in welcher $R^6$, $R^7$, $R^8$, $R^9$ und $R^{10}$ wie im Anspruch 1 definiert sind und $X^2$ im oben genannten Fall i. -SH, $-S^-$ oder $-SO_2^-$ und

im oben genannten Fall ii. eine Abgangsgruppe bedeutet, oder

b) eine Verbindung der Formel IV,

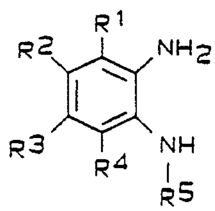

(IV)

in welcher $R^1$, $R^2$, $R^3$, $R^4$ und $R^5$ wie oben definiert sind,

umsetzt mit einer Verbindung der Formel V

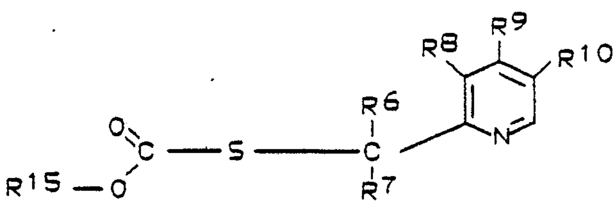

(V)

in welcher $R^6$, $R^7$, $R^8$, $R^9$ und $R^{10}$ wie oben definiert sind und $R^{15}$ für eine veresternde Gruppe steht, oder

c) eine Verbindung der Formel VI

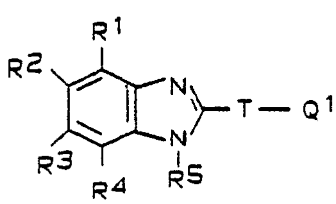

(VI)

in welcher $R^1$, $R^2$, $R^3$, $R^4$ und T wie in Anspruch 1 definiert sind,

$R^5$ Wasserstoff oder eine Schutzgruppe bedeutet und
$Q^1$ a) für $CR^6R^7$-M steht oder
b) eine Abgangsgruppe bedeutet,
umsetzt mit einer Verbindung der Formel VII

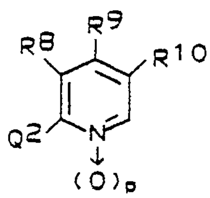

(VII)

in welcher $R^8$ $R^9$ und $R^{10}$ wie in Anspruch 1 definiert sind und
$Q^2$ a) falls $Q^1$ wie oben unter a) definiert ist, eine Abgangsgruppe bedeutet, oder
b) falls $Q^1$ wie oben unter b) definiert ist, für $CR^6R^7$-M steht, und
p = 0 oder 1 ist,
wobei in den Formeln VI und VII $R^6$ und $R^7$ wie in Anspruch 1 definiert ist und
M für ein Alkalimetallatom oder dessen Äquivalent steht,
i. in einer Verbindung der Formel I (eine) gegebenenfalls vorhandene -S-Gruppe(n) gewünschtenfalls zu(r) -SO- oder -$SO_2$-Gruppe(n) oxidiert,
ii. in einer Verbindung der Formel I (eine) gegebenenfalls vorhandene -SO-Gruppe(n) gewünschtenfalls zu(r) -$SO_2$-Gruppe(n) oxidiert,
iii. eine Verbindung der Formel I, worin $R^5$ für Wasserstoff steht, gewünschtenfalls acyliert, alkyliert oder aralkyliert,
iv. in einer Verbindung der Formel I, worin $R^5$ eine Schutzgruppe bedeutet, gewünschtenfalls diese unter Bildung einer Verbindung der Formel I mit $R^5$ = Wasserstoff abspaltet,
v. eine Verbindung der Formel I gewünschtenfalls in ihre physiologisch verträglichen Salze überführt,
wobei zwei oder mehr der Maßnahmen i.-iv auch in einer anderen als der angegebenen Reihenfolge ausgeführt werden können.

7. Verbindung gemäß einem der Ansprüche 1 bis 5 zur Anwendung als Heilmittel.

8. Verbindung gemäß einem der Ansprüche 1 bis 5 zur Anwendung als Magensäuresekretionshemmer, als Heilmittel bei der Behandlung entzündlicher Darmerkrankungen und/oder als Heilmittel bei der Behandlung der Diarrhoe.

9. Mittel enthaltend eine Verbindung gemäß einem der Ansprüche 1 bis 5.

10. Verfahren zur Herstellung eines Mittels gemäß Anspruch 9 dadurch gekennzeichnet, daß man eine Verbindung gemäß einem der Ansprüche 1 bis 5 in keine geeignete Darreichungsform bringt.

Patentansprüche für Vertragsstaaten folgende: ES, GR

1. Verfahren zur Herstellung einer Verbindung der Formel I

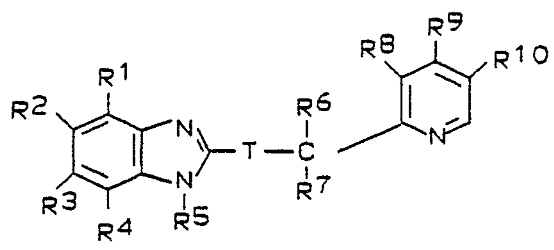

(I)

in welcher
T -S-, -SO- oder -$SO_2$- bedeutet,
$R^1$, $R^2$, $R^3$ und $R^4$ gleich oder verschieden sind und Wasserstoff, Halogen, Cyano, Nitro, ($C_1$-$C_6$)-Alkyl, einen halogenierten Alkylrest $(C_fH_{2f+1-g})Hal_g$, ($C_1$-$C_6$)-Hydroxyalkyl, ($C_1$-$C_6$)-Alkoxy, einen halogenierten Alkoxyrest $OC_fH_{(2f+1-g)}Hal_g$, ($C_1$-$C_6$)-Alkylmercapto, ($C_1$-$C_6$)-Alkylsulfinyl, ($C_1$-$C_6$)-Alkylsulfonyl, ($C_1$-$C_6$)-Alkylcarbonyl, ($C_1$-$C_6$)-Alkoxycarbonyl, Carbamoyl, N-($C_1$-$C_4$)-Alkylcarbamoyl, N,N-Di-($C_1$-$C_4$)-alkylcarbamoyl, ($C_1$-$C_6$)-Alkylcarbonyloxy, ($C_3$-$C_8$)-Cycloalkyl, Phenyl, Benzyl, Phenoxy, Benzyloxy, Anilino, N-methylanilino,

Phenylmercapto, Phenylsulfonyl, Phenylsulfinyl, Sulfamoyl, N-($C_1$-$C_4$)-Alkylsulfamoyl oder N,N-Di-($C_1$-$C_4$)-alkylsulfamoyl bedeuten, oder zwei der Reste $R^1$, $R^2$, $R^3$ und $R^4$ die benachbart sind, gemeinsam für --[$CQ_2$]$_n$ oder -CH=CH-CH=CH- stehen, worin eine oder zwei $CQ_2$-Gruppen durch gleiche oder verschiedene Reste aus der Reihe O, S, SO und $SO_2$ ersetzt sein können, und die übrigen der Reste $R^1$, $R^2$, $R^3$ und $R^4$ vorstehende Bedeutung haben;

$R^5$ Wasserstoff, ($C_1$-$C_{11}$-Alkanoyl, ($C_1$-$C_6$)-Alkylcarbamoyl oder eine andere physiologisch verträgliche, vorzugsweise im sauren Medium und/oder unter physiologischen Bedingungen abspaltbare $N^{im}$-Schutzgruppe bedeutet;

$R^6$ und $R^7$ gleich oder verschieden sind und Wasserstoff oder ($C_1$-$C_3$)-Alkyl bedeuten;

$R^8$ und $R^{10}$ gleich oder verschieden sind und Wasserstoff, Halogen, ($C_1$-$C_{12}$)-Alkyl, Trifluormethyl, ($C_3$-$C_6$)-Cycloalkyl, ($C_1$-$C_{12}$)-Alkoxy, -O-$CH_2$-$C_fH_{(2f+1-g)}F_g$, -NR′R″, ($C_1$-$C_{12}$)-Alkoxy-($C_1$-$C_{12}$)-alkyl, ($C_1$-$C_{12}$)-Alkoxy-($C_1$-$C_{12}$)-alkoxy, ($C_7$-$C_{11}$)-Aralkyloxy, ($C_1$-$C_{12}$)-Alkylmercapto, ($C_1$-$C_{12}$)-Alkylsulfinyl oder ($C_1$-$C_{12}$)-Alkylsulfonyl bedeuten, mit der Maßgabe, daß mindestens einer der Reste $R^8$ und $R^{10}$ Halogen bedeuten muß;

$R^9$ ($C_6$-$C_{12}$)-Alkoxy, ($C_3$-$C_8$)-Cycloalkyloxy, ($C_7$-$C_{11}$)-Aralkyloxy, ($C_1$-$C_{12}$)-Alkylmercapto, ($C_1$-$C_{12}$)-Alkylsulfinyl oder ($C_2$-$C_{12}$)-Alkylsulfonyl bedeutet;

Hal Halogen bedeutet;

Q Wasserstoff oder Halogen bedeutet;

R′ und R″ gleich oder verschieden sind und Wasserstoff oder ($C_1$-$C_4$)-Alkyl bedeuten oder

R′ und R″ gemeinsam für -[$CH_2$]$_h$- stehen, worin eine $CH_2$-Gruppe durch O, S, N-($C_1$-$C_4$)-Alkanoylimino oder N-($C_1$-$C_4$)-Alkoxycarbonylimino ersetzt sein kann;

f = 1, 2, 3, 4, 5 oder 6, vorzugsweise 1-4 ist;

g = 1 bis (2f+1) ist;

h = 4, 5 oder 6 ist;

und

n = 3 oder 4 ist,

oder deren physiologisch verträglichen Salzen, dadurch gekennzeichnet, daß man

a) eine Verbindung der Formel II

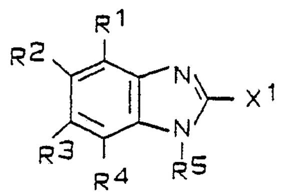

(II)

in welcher $R^1$, $R^2$, $R^3$, $R^4$ und $R^5$ wie im Anspruch 1. definiert sind und

$X^1$ i. eine Abgangsgruppe oder

ii. -SH, $-S^-$ oder $-SO_2^-$ bedeutet,

umsetzt mit einer Verbindung der Formel III

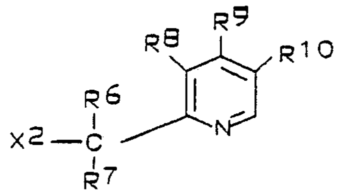

(III)

in welcher $R^6$, $R^7$, $R^8$ $R^9$ und $R^{10}$ wie im Anspruch 1 definiert sind und

$X^2$ im oben genannten Fall i. -SH, $-S^-$ oder $-SO_2^-$ und

im oben genannten Fall ii. eine Abgangsgruppe bedeutet, oder

b) eine Verbindung der Formel IV,

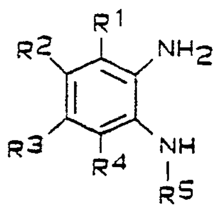

(IV)

in welcher $R^1$, $R^2$, $R^3$, $R^4$ und $R^5$ wie oben definiert sind, umsetzt mit einer Verbindung der Formel V

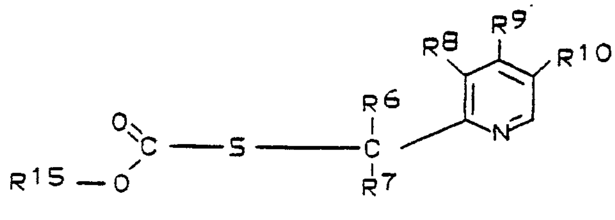

(V)

in welcher $R^6$, $R^7$, $R^8$, $R^9$ und $R^{10}$ wie oben definiert sind und $R^{15}$ für eine veresternde Gruppe steht, oder

c) eine Verbindung der Formel VI

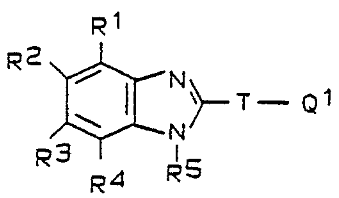

(VI)

in welcher $R^1$, $R^2$, $R^3$, $R^4$ und T wie in Anspruch 1 definiert sind,
$R^5$ Wasserstoff oder eine Schutzgruppe bedeutet und
$Q^1$ a) für $CR^6R^7$-M steht oder
b) eine Abgangsgruppe bedeutet,
umsetzt mit einer Verbindung der Formel VII

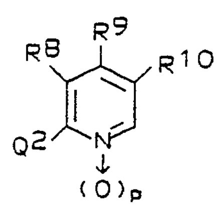

(VII)

in welcher $R^8$, $R^9$ und $R^{10}$ wie in Anspruch I definiert sind und
$Q^2$ a) falls $Q^1$ wie oben unter a) definiert ist, eine Abgangsgruppe bedeutet, oder
b) falls $Q^1$ wie oben unter b) definiert ist, für $CR^6R^7$-M steht, und
p = 0 oder 1 ist,
wobei in den Formeln VI und VII $R^6$ und $R^7$ wie in Anspruch 1 definiert ist und
M für ein Alkalimetallatom oder dessen Äquivalent steht,
i. in einer Verbindung der Formel I (eine) gegebenenfalls vorhandene -s-Gruppe(n) gewünschtenfalls zu(r) -SO- oder $-SO_2$-Gruppe(n) oxidiert,
ii. in einer Verbindung der Formel I (eine) gegebenenfalls vorhandene -SO-Gruppe(n) gewünschtenfalls zu(r) $-SO_2$-Gruppe(n) oxidiert,
iii. eine Verbindung der Formel I, worin $R^5$ für Wasserstoff steht, gewünschtenfalls acyliert, alkyliert oder aralkyliert,
iv. in einer Verbindung der Formel I, worin $R^5$ eine Schutzgruppe bedeutet, gewünschtenfalls diese unter Bildung einer Verbindung der Formel I mit $R^5$ = Wasserstoff abspaltet,

v. eine Verbindung der Formel I gewünschtenfalls in ihre physiologisch verträglichen Salze überführt, wobei zwei oder mehr der Maßnahmen i.-iv. auch in einer anderen als der angegebenen Reihenfolge ausgeführt werden können.

2. Verfahren gemäß Anspruch 1, dadurch gekennzeichnet, daß eine Verbindung der Formel I hergestellt wird, in welcher T für -SO- steht oder deren physiologisch verträglichen Salze.

3. Verfahren gemäß einem oder mehrerer der Ansprüche 1 bis 2, dadurch gekennzeichnet, daß eine Verbindung der Formel I hergestellt wird, in welcher $R^8$ und $R^{10}$ gleich oder verschieden sind und Wasserstoff, Halogen oder $(C_1-C_6)$-Alkyl bedeuten, wobei mindestens einer dieser Reste Halogen bedeuten muß,oder deren physiologisch verträglichen Salze.

4. Verfahren gemäß einem oder mehrerer der Ansprüche 1 bis 3, dadurch gekennzeichnet, daß eine Verbindung der Formel I hergestellt wird, in welcher
T eine -SO-Gruppe bedeutet;
$R^2$, $R^3$, $R^6$ und $R^7$ jeweils Wasserstoff bedeuten;
$R^9$ $(C_1-C_{12})$-Alkoxy, $-O-CH_2-C_fH_{(2f+1-g)}F_g$, $(C_1-C_{12})$-Alkoxy-$(C_1-C_{12})$-alkyl, $(C_1-C_{12})$-Alkoxy-$(C_1-C_{12})$-alkoxy, $(C_7-C_{11})$Aralkyloxy, $(C_1-C_{12})$-Alkylmercapto, $(C_1-C_{12})$-Alkylsulfinyl oder $(C_1-C_{12})$-Alkylsulfonyl bedeuten;
$R^8$ und $R^{10}$ gleich oder verschieden sind und Wasserstoff, Halogen oder $(C_1-C_6)$-Alkyl bedeuten, wobei mindestens einer der Reste $R^8$ und $R^{10}$ Halogen bedeuten muß, oder deren physiologisch verträglichen Salze.

5. Verfahren gemäß einem oder mehrerer der Ansprüche 1 bis 4, dadurch gekennzeichnet, daß man eine Verbindung der Formel I herstellt, in welcher $R^5$ Wasserstoff bedeutet, oder deren physiologisch verträglichen Salze.

6. Verfahren zur Herstellung eines Mittels enthaltend eine Verbindung gemäß einem der Ansprüche 1 bis 5, dadurch gekennzeichnet, daß man eine Verbindung der Formel I in eine geeignete Darreichungsform bringt.

Europäisches Patentamt

# EUROPÄISCHER RECHERCHENBERICHT

Nummer der Anmeldung

EP 88 11 0774

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 093 252 (L. HEUMANN)<br>* Insgesamt *<br>--- | | C 07 D 401/12<br>A 61 K 31/415 |
| A | EP-A-0 176 308 (THE UPJOHN CO.)<br>* Insgesamt *<br>--- | | |
| D,A | EP-A-0 184 322 (SMITH KLINE & FRENCH)<br>* Insgesamt *<br>--- | | |
| P,A | EP-A-0 220 053 (FISONS)<br>* Insgesamt *<br>--- | | |
| P,A | EP-A-0 246 774 (SITH KLINE & FRENCH)<br>* Insgesamt *<br>----- | | |
| | | | RECHERCHIERTE SACHGEBIETE (Int. Cl.4) |
| | | | C 07 D 401/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 10-10-1988 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)